(19) Europäisches Patentamt
European Patent Office
Office européen des brevets

(11) EP 1 705 227 A2

(12) # EUROPÄISCHE PATENTANMELDUNG

(43) Veröffentlichungstag:
**27.09.2006 Patentblatt 2006/39**

(21) Anmeldenummer: **06001573.2**

(22) Anmeldetag: **26.01.2006**

(51) Int Cl.:
***C09B 7/00*** (2006.01) ***C09B 57/04*** (2006.01)
***A61Q 5/10*** (2006.01) ***A61K 8/49*** (2006.01)

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **14.03.2005 DE 102005011924**

(71) Anmelder: **Henkel Kommanditgesellschaft auf Aktien**
**40589 Düsseldorf (DE)**

(72) Erfinder:
- **Naumann, Frank**
**40589 Düsseldorf (DE)**
- **Rose, David**
**40723 Hilden (DE)**

(54) **Neue Farbstoffe und Färbemittel mit speziellen Carbonylverbindungen**

(57) Hybridfarbstoffe der Struktur (I),

X - S - Y (I)

in der X steht für eine Gruppe, die abgeleitet ist von
- Indolin-2,3-dion (Isatin) oder substituierten, insbesondere N-substituierten Indolin-2,3-dionen (Isatinen),
- 2-Oxoessigsäure oder 2- Oxoessigsäurederivaten,
- Tropolon oder Tropolonderivaten,
- ungesättigten Aldehyden;
Y steht für eine Gruppe, die abgeleitet ist von
- einem Oxidationsfarbstoffvorprodukt vom Kuppler- oder Entwicklertyp,
- einem Derivat des Indols oder Indolins als Vorläufer des Melanins oder
- einem direktziehenden Farbstoff, und
S steht für eine direkte Bindung oder eine Spacer-Gruppe

mit der Maßgabe, daß S nicht für eine Alkylen-, Mono- oder Poly-hydroxyalkylengruppe steht, wenn Y von einem direktziehenden Farbstoff abgeleitet ist, eignen sich hervorragend zum Färben keratinischer Fasern, insbesondere menschlicher Haare. Durch Verwendung von Färbemitteln mit diesen Farbstoffen lassen sich eine Reihe von Problemen, die bei Verwendung komplexer Farbstoffmischungen auftreten, umgehen oder deutlich minimieren.

EP 1 705 227 A2

## Beschreibung

Die Erfindung betrifft neue Farbstoffe und Farbstoffvorprodukte, die insbesondere zum Färben von Keratinfasern geeignet sind, die Verwendung dieser Farbstoffe und Farbstoffvorprodukte sowie diese Farbstoffe und/oder Farbstoffvorprodukte enthaltende Färbemittel.

Im Rahmen der Produkte, die zur kosmetischen Behandlung des menschlichen Körpers bereitgestellt werden, spielen Mittel zur Veränderung oder Nuancierung der Farbe des Kopfhaares eine herausragende Rolle. Sieht man von den Blondiermitteln, die eine oxidative Aufhellung der Haare durch Abbau der natürlichen Haarfarbstoffe bewirken, ab, so sind im Bereich der Haarfärbung im wesentlichen drei Typen von Haarfärbemitteln von Bedeutung:

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe mehr benötigen. Zu diesen Farbstoffen gehört beispielsweise das bereits aus dem Altertum zur Färbung von Körper und Haaren bekannte Henna.

Weiterhin hat in jüngster Zeit ein neuartiges Färbeverfahren große Beachtung gefunden. Bei diesem Verfahren werden Vorstufen des natürlichen Haarfarbstoffes Melanin, insbesondere Derivate des Indols oder Indolins, auf das Haar aufgebracht und bilden im Rahmen oxidativer Prozesse im Haar praktisch naturidentische Farbstoffe aus. Ein solches Verfahren mit 5,6-Dihydroxyindolinen als Farbstoffvorprodukte wurde in der EP-B1-530 229 beschrieben. Bei, insbesondere mehrfacher, Anwendung von Mitteln mit 5,6-Dihydroxyindolin ist es möglich, Personen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß.

Zwar ist es prinzipiell möglich, Färbemittel mit lediglich einem Farbstoff oder einer Farbstoffvorstufe zu formulieren. Mit Ausnahme weniger Produkte, die z. B. Vorläufer des Melanins enthalten, haben solche Färbemittel in der Praxis aber nur geringe Bedeutung erlangt.

Kommerzielle Haarfärbeprodukte enthalten vielmehr üblicherweise eine Mischung von etwa 3 bis 8 unterschiedlichen Farbstoffen und/oder Farbstoffvorprodukten. Die einzelnen Farbstoffe weisen aber in der Regel unterschiedliche Aufziehvermögen, Licht-, Schweiß-, Reib- und Waschechtheiten auf, die zudem noch stark von den strukturellen Eigenschaften und dem Pflegezustand des Haares abhängig sein können. Diese Unterschiede sind vor allem dann ausgeprägt, wenn, wie für viele Nuancen bisher unverzichtbar, direktziehende Farbstoffe zur Einstellung der Nuance in Oxidationsfärbemitteln eingesetzt werden.

Es bedarf daher bei der Entwicklung neuer Haarfärbemittel häufig umfangreicher Versuche, nicht nur um bestimmte Nuancen zu erzielen, sondern vor allem um sicherzustellen, daß die Färbung über den gewünschten Zeitraum sowohl in der Nuance als auch in der Intensität stabil ist.

Es wurde nunmehr überraschenderweise gefunden, daß viele der oben genannten Probleme ganz oder zumindest teilweise vermieden werden können, wenn Substanzen eingesetzt werden, die sowohl über die Eigenschaften eines direktziehenden Farbstoffes als auch über die Eigenschaften eines Oxidationsfarbstoffvorproduktes, eines Vorläufers des Melanins oder eines weiteren direktziehenden Farbstoffes verfügen. Insbesondere hat sich gezeigt, daß die Farbstoffe über ein sehr gutes, mit bekannten Haarfarbstoffen bzw. Haarfarbstoffvorprodukten vergleichbares Aufziehvermögen auf das Haar verfügen und zu brillanten, intensiven Haarfärbungen führen. Aufgrund der molekularen Verknüpfung läßt sich so das Problem unterschiedlicher Echtheitseigenschaften der beiden Farbstoffe bzw. Farbstoffvorprodukte in vielen Fällen weitgehend überwinden.

Solche Substanzen, die im weiteren als "Hybridfarbstoffe" bezeichnet werden, sind beispielsweise in der DE 199 30 927 A1 beschrieben. Die dort offenbarten Hybridfarbstoffe, besitzen die Struktur

$$X - S - Y$$

in der
X steht für eine Gruppe, die abgeleitet ist von einem direktziehenden Farbstoff,

Y steht für eine Gruppe, die abgeleitet ist von

- einem Oxidationsfarbstoffvorprodukt vom Kuppler- oder Entwicklertyp,
- einem Derivat des Indols oder Indolins als Vorläufer des Melanins oder
- einem direktziehenden Farbstoff, und

S steht für eine direkte Bindung oder eine Spacer-Gruppe mit der Maßgabe, daß S nicht für eine Alkylen-, Mono- oder Polyhydroxyalkylengruppe steht, wenn Y von einem direktziehenden Farbstoff abgeleitet ist.

Es bestand die Aufgabe, die Farbstoffklasse der Hybridfarbstoffe weiterzuentwickeln und neue, farbechte und brillante Farbstoffe für Färbungen von Keratinfasern bereitzustellen.

Es wurde nun gefunden, daß Hybridfarbstoffe der genannten Strukturformel, in denen die Gruppe X aus bestimmten Carbonylverbindungen ausgewählt ist, neuartige, gegen Licht und Sauerstoff beständige und brillante Färbungen liefern.

Ein erster Gegenstand der vorliegenden Erfindung ist daher ein Hybridfarbstoff, insbesondere zum Färben keratinischer Fasern, der Struktur (I),

$$X - S - Y \quad (I)$$

in der X steht für eine Gruppe, die abgeleitet ist von

- Indolin-2,3-dion (Isatin) oder substituierten, insbesondere N-substituierten Indolin-2,3-dionen (Isatinen),
- 2-Oxoessigsäure oder 2- Oxoessigsäurederivaten,
- Tropolon oder Tropolonderivaten,
- ungesättigten Aldehyden;
  Y steht für eine Gruppe, die abgeleitet ist von
- einem Oxidationsfarbstoffvorprodukt vom Kuppler- oder Entwicklertyp,
- einem Derivat des Indols oder Indolins als Vorläufer des Melanins oder
- einem direktziehenden Farbstoff, und
  S steht für eine direkte Bindung oder eine Spacer-Gruppe mit der Maßgabe, daß S nicht für eine Alkylen-, Mono- oder Poly-hydroxyalkylengruppe steht, wenn Y von einem direktziehenden Farbstoff abgeleitet ist.

Die Verbindungen gemäß Struktur (I) sind mit üblichen Syntheseverfahren der organischen Chemie zugänglich.

In den erfindungsgemäßen Hybridfarbstoffen steht X für eine Gruppe, die abgleitet ist von

- Indolin-2,3-dion (Isatin) oder substituierten, insbesondere N-substituierten Indolin-2,3-dionen (Isatinen),
- 2-Oxoessigsäure oder 2- Oxoessigsäurederivaten,
- Tropolon oder Tropolonderivaten,
- ungesättigten Aldehyden.

Diese Gruppen und bevorzugte Vertreter aus ihnen werden nachfolgend beschrieben.

X kann für Isatin oder substituierte, insbesondere N-substituierte Isatinen stehen.

Die substituierten Isatine sind entweder literaturbekannte Verbindungen oder lassen sich durch literaturbekannte Standardsynthesen darstellen, u. a. durch Reaktion von Isatin bzw. im Benzolkern substituierten Isatin-Derivaten oder ihren Natriumsalzen mit entsprechenden Halogen- oder Epoxyverbindungen.

Besonders bevorzugte Vertreter aus der Gruppe der (gegebenenfalls substituierten) Isatine werden nachstehend genannt. Hier sind erfindungsgemäße Hybridfarbstoffe bevorzugt, bei denen X für eine Gruppe steht, die ausgewählt ist aus

- Indolin-2,3-dion,
- 1-(4-Nitrophenyl)indolin-2,3-dion,
- 1-(2-Nitrophenyl)indolin-2,3-dion,
- 1-(4-Amino-2-nitrophenyl)indolin-2,3-dion,
- 1-(5-Nitro-2-pyridyl)indolin-2,3-dion,
- 1-Hydroxymethylisatin,
- 1-Hydroxymethyl-5-methylisatin,

- 1-Hydroxymethyl-5-chlorisatin,
- 1-Hydroxymethyl-5-sulfoisatin,
- 1-Hydroxymethyl-5-carboxyisatin,
- 1-Hydroxymethyl-5-nitroisatin,
- 1-Hydroxymethyl-5-bromisatin,
- 1-Hydroxymethyl-5-methoxyisatin,
- 1-Hydroxymethyl-5,7-dichlorisatin,
- 1-Dimethylaminomethylisatin,
- 1-Diethylaminomethylisatin,
- 1-(2,3-Dihydroxypropyl)isatin,
- 1-(Bis-(2-hydroxyethyl)-aminomethyl)-isatin,
- 1-(2-hydroxyethylaminomethyl)-isatin,
- 1-(Bis-(2-hydroxypropyl)-aminomethyl)-isatin,
- 1-Pyrrolidinomethylisatin,
- 1-Piperidinomethylisatin,
- 1-Morpholinomethylisatin,
- 1-(2-Morpholinoethyl)isatin,
- 1-(1,2,4-Triazolyl)-methylisatin,
- 1-(1-Imidazolyl)-methylisatin,
- 1-Carboxymethylaminomethylisatin,
- 1-(2-Carboxyethylaminomethyl)-isatin,
- 1-(3-Carboxypropylaminomethyl)-isatin,
- 1-(Bis-(2-hydroxyethyl)-aminomethyl)-5-methylisatin,
- 1-Piperidinomethyl-5-chlorisatin,
- 1-(2-Sulfoethylaminomethyl)-isatin,
- 1-(2-Sulfoethyl)isatin,
- 1-(3-Sulfopropyl)isatin,
- 1-Allylisatin,
- 1-(2-Dimethylamino)isatin,
- 1-(2-Pyrrolidino)isatin,
- 1-(2-Piperidino)isatin,
- N-(2-furylmethyl)isatin,
- 1-(2-Thienylmethyl)isatin,
- 1-(2-, N- (3- oder N-(4-Pyridylmethyl)-isatin,
- 1-Allylisatin-5-sulfonsäure,
- 5-Chlorisatin,
- 5-Methyl-N-(hydroxyethyl)isatin,
- 5,7-Dichlorisatin,
- 5-Nitro-N-allylisatin.

X kann auch für 2-Oxoessigsäure oder 2- Oxoessigsäurederivate stehen. Besonders bevorzugte Vertreter aus der Gruppe der 2- Oxoessigsäurederivate werden nachstehend genannt. Hier sind erfindungsgemäße Hybridfarbstoffe bevorzugt, bei denen X für eine Gruppe steht, die ausgewählt ist aus

- 2-Oxoessigsäure,
- 2-(2-Aminophenyl)-2-Oxoessigsäure,
- 2-(2-Amino-5-methylphenyl)-2-Oxoessigsäure,
- 2-(2-Amino-5-chlorphenyl)-2-Oxoessigsäure,
- 2-(2-Amino-5- nitrophenyl)-2-Oxoessigsäure,
- 2-(2-Amino- 5-methoxyphenyl)-2-Oxoessigsäure,
- 2-(2-Hydroxylaminophenyl)-2-Oxoessigsäure,
- 2-(2-(2-Hydroxylethylamino)-phenyl)-2-Oxoessigsäure,
- 2-(2-Benzylaminophenyl)-2-Oxoessigsäure,
- 2-(2-(2- Sulfoehtylamino)-phenyl)-2-Oxoessigsäure,
- 2-(2-(3-Sulfopropylamino)-phenyl)-2-Oxoessigsäure,
- 2-(2- Allylaminophenyl)-2-Oxoessigsäure,
- 2-(2- Carboxymethylaminophenyl)-2-Oxoessigsäure,
- 2-(2-Amino-5- bromphenyl)-2-Oxoessigsäure,

- 2-(2-Amino-5,7-dichlorphenyl)-2-Oxoessigsäure,
- 2-(2-Amino-5-sulfophenyl)-2-Oxoessigsäure,
- 2-(2-Amino-5-carboxyphenyl)-2-Oxoessigsäure,
- 2-(2-Amino-4-carboxyphenyl)-2-Oxoessigsäure,
- sowie Natrium-, Kalium-, Ammonium- oder Calciumsalze der vorstehend genannten Verbindungen.

X kann auch für Tropolon oder Tropolonderivate stehen. Besonders bevorzugte Vertreter aus der Gruppe der Tropolonderivate werden nachstehend genannt.

Hier sind erfindungsgemäße Hybridfarbstoffe bevorzugt, bei denen X für eine Gruppe, die ausgewählt ist aus Tropolonen der Formel II

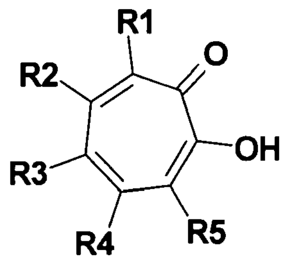

(II)

in der $R^1$, $R^2$, $R^3$, $R^4$, und $R^5$ ein Wasserstoff-, ein Halogenatom oder eine ($C_1$-$C_4$)-Alkylgruppe bedeuten, wobei die Reste $R^1$ und $R^2$ auch gemeinsam einen an die Doppelbindung ankondensierten Benzolring, der seinerseits durch Hydroxy-, ($C_1$-$C_4$)-Alkyl-, ($C_1$-$C_4$)-Alkoxygruppen oder Halogenatome substituiert sein kann, bilden können, und wobei folgende Verbindungen besonders bevorzugt sind:

- Tropolon,
- Purpurogallin,
- der Methylether von Purpurogallin,
- 3,5,7-Tribromtropolon,
- 3,5,7-Trimethyltropolon,
- 7-(2-Propyl)-tropolon.

X kann auch für ungesättigte Aldehyde stehen. Besonders bevorzugte Vertreter aus der Gruppe der ungesättigten Aldehyde werden nachstehend genannt. Bevorzugt erfindungsgemäße Hybridfarbstoffe aus dieser Gruppe sind dadurch gekennzeichnet, daß X steht für eine Gruppe, die ausgewählt ist aus ungesättigten Aldehyden beschrieben durch die tautomeren Grenzformeln IIIa bzw. IIIb,

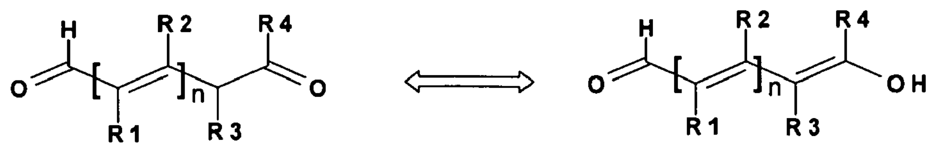

(IIIa) (IIIb)

in der $R^1$, $R^2$, $R^3$ und $R^4$ unabhängig voneinander für Wasserstoff, Halogen, einen $C_1$-$C_4$-Alkoxy-, $C_1$-$C_4$-Alkyl-, Aryl- oder $C_1$-$C_4$-Alkoxy-$C_1$-$C_4$-Alkylrest und n für die Zahlen 1 oder 2 stehen, wobei $R^1$ und $R^2$, $R^1$ und $R^3$, $R^2$ und $R^3$ sowie $R^2$ und $R^4$ jeweils zusammen mit dem Restmolekül einen 5- bis 7-gliedrigen Ring bilden können, wenn n gleich 1 ist, sowie den entsprechenden Mono-, Bis- bzw. ω-Alkoxyacetalen, wobei folgende Verbindungen besonders bevorzugt sind:

- Glutaconaldehyd,

- 1-Formyl-3-hydroxymethylen-I-cyclohexen,
- 1-Formyl-3-hydroxymethylen-1-cyclohepten,
- 7-Hydroxy-2,4,6-heptatrienal
- substituierten Derivate sowie physiobiologisch verträgliche Salze der vorstehend genannten Verbindungen, insbesondere Alkali- und Ammoniumsalzen, wobei die Tetrabutylammoniumsalze und Na-Salze sowie deren Gemische besonders bevorzugt sind.

Die Gruppe Y steht für eine Gruppe, die abgeleitet ist von

- einem Oxidationsfarbstoffvorprodukt vom Kuppler- oder Entwicklertyp,
- einem Derivat des Indols oder Indolins als Vorläufer des Melanins oder
- einem direktziehenden Farbstoff.

Gemäß einer ersten, bevorzugten, Ausführungsform der vorliegenden Erfindung leitet sich die Gruppe Y ab von einem Oxidationsfarbstoffvorprodukt vom Kupplertyp.

Bevorzugte Klassen von Oxidationsfarbstoffvorprodukten vom Kupplertyp, von denen sich die Gruppe Y ableiten kann, sind

- 3-Aminophenol und dessen Derivate
  Bevorzugte Vertreter sind 5-Amino-2-methylphenol, 5-(3-Hydroxypropylamino)-2-methylphenol, 3-Amino-2-chlor-6-methylphenol, 2-Hydroxy-4-aminophenoxy-ethanol, 3-Amino-6-methoxy-2-methylaminophenol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-Dimethylamino-phenol, 3-Diethylamino-phenol, N-Cyclopentyl-3-aminophenol, 1,3-Dihydroxy-5-(methyl-amino)-benzol, 3-Ethylamino-4-methylphenol und 2,4-Dichlor-3-aminophenol.
- 2-Aminophenol und dessen Derivate
- 1,3-Diaminobenzol und dessen Derivate
  Bevorzugte Vertreter sind 2,4-Diamino-phenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol, 1-Amino-3-bis-(2'-hydroxyethyl)-aminobenzol, 1,2-Bis-(2,4-diaminophenoxy)-benzol und 1,3-Bis-(2,4-diaminophenoxy)-benzol.
- 1,2-Diaminobenzol und dessen Derivate
  Bevorzugte Vertreter sind 3,4-Diaminobenzoesäure und 2,3-Diamino-1-methyl-benzol.
- Di- und Trihydroxybenzole und deren Derivate
  Bevorzugte Vertreter sind Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol sowie weiterhin Resorcindimethylether.
- Pyridinderivate
  Bevorzugte Vertreter sind 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-3,4-diaminopyridin, 2,6-Dihydroxy-4-methylpyridin, 2,6-Diaminopyridin, 2,3-Diamino-6-methoxypyridin und 3,5-Diamino-2,6-dimethoxypyridin.
- Naphthalinderivate
  Bevorzugte Vertreter sind 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin sowie weiterhin 1-Aminonaphthalin.
- Morpholinderivate
  Bevorzugte Vertreter sind 6-Hydroxybenzomorpholin und 6-Aminobenzomorpholin.
- Chinoxalinderivate
  Ein bevorzugter Vertreter ist 6-Methyl-1,2,3,4-tetrahydrochinoxalin.
- Pyrazolderivate
  Ein bevorzugter Vertreter ist 1-Phenyl-3-methylpyrazol-5-on.
- Indolderivate
  Bevorzugte Vertreter sind 4-Hydroxyindol, 6-Hydroxyindol und 7-Hydroxyindol.
- Methylendioxybenzolderivate
  Bevorzugte Vertreter sind 3,4-Methylendioxyphenol, 1-Amino-3,4-methylendioxybenzol und 1-(2'-Hydroxyethyl)-amino-3,4-methylendioxybenzol.

Bezüglich weiterer Oxidationsfarbstoffvorprodukte vom Kupplertyp, von denen sich die Gruppe Y ableiten kann, wird weiterhin ausdrücklich auf die bekannten Monographien, z. B. Ch. Zviak, The Science of Hair Care, Kapitel 8 (Seiten 264-267), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Gemäß einer zweiten, ebenfalls bevorzugten, Ausführungsform der vorliegenden Erfindung leitet sich die Gruppe Y ab von einem Oxidationsfarbstoffvorprodukt vom Entwicklertyp.

Bevorzugte Klassen von Oxidationsfarbstoffvorprodukten vom Entwicklertyp, von denen sich die Gruppe Y ableiten kann, sind

- 1,4-Diaminobenzol und dessen Derivate
  Bevorzugte Vertreter sind p-Phenylendiamin, p-Toluylendiamin, 1-(2'-Hydroxy-ethyl)-2,5-diaminobenzol, N,N-Bis-(2-hydroxy-ethyl)-p-phenylendiamin, 2-(2,5-Diaminophenoxy)-ethanol, 1,3-Bis(N(2-hydroxyethyl)-N(4-aminophenylamino))-2-propanol, 1,10-Bis-(2,5-diaminophenyl)-1,4,7,10-tetraoxadecan und 1,4-Bis-(4-aminophenyl)-diazacycloheptan sowie entsprechende Verbindungen mit einem oder mehreren Halogenatomen, insbesondere Chlor und Fluor, am Benzolring.
- 1,2-Diaminobenzol und dessen Derivate
- 4-Aminophenol und dessen Derivate
  Bevorzugte Vertreter sind p-Aminophenol, 2-Chlor-4-aminophenol, 4-Amino-3-methylphenol, 2-Hydroxyethylamino-4-amino-phenol, 4,4'-Diaminodiphenylamin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 4-Amino-2-(2-hydroxyethoxy)-phenol.
- 2-Aminophenol und dessen Derivate
  Ein bevorzugter Vertreter ist o-Aminophenol.
- Diaminopyridinderivate
- heterocyclische Hydrazone
- 4-Aminopyrazolderivate
  Bevorzugte Vertreter sind 1-Phenyl-3-carboxyamido-4-amino-pyrazolon-5 sowie 4,5-Diaminopyrazol-Derivate nach EP 0 740 931 bzw. WO 94/08970 wie z. B. 4,5-Diamino-1-(2'-hydroxyethyl)-pyrazol.
- Pyrimidin-Derivate
  Bevorzugte Vertreter sind 2,4,5,6-Tetraaminopyrimidin, 2-Hydroxy-4,5,6-triamino-pyrimidin, 4-Hydroxy-2,5,6-triaminopyrimidin, 2,4-Dihydroxy-5,6-diamino-pyrimidin und 2-Dimethylamino-4,5,6-triaminopyrimidin.

Bezüglich weiterer Oxidationsfarbstoffvorprodukte vom Entwicklertyp, von denen sich die Gruppe Y ableiten kann, wird weiterhin ausdrücklich auf die bekannten Monographien, z. B. Ch. Zviak, The Science of Hair Care, Kapitel 8 (Seiten 264-267), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Gemäß einer dritten, bevorzugten, Ausführungsform der vorliegenden Erfindung leitet sich die Gruppe Y ab von einem Vorläufer des Melanins, ausgewählt aus den Derivaten des Indols und Indolins. Im Rahmen der vorliegenden Erfindung werden unter "Vorläufer des Melanins" solche Derivate des Indol und des Indolins verstanden, die im Rahmen eines oxidativen Prozesses in der Lage sind, Melaninfarbstoffe oder entsprechende Melaninfarbstoff-Derivate auszubilden.

Erfindungsgemäß bevorzugt leiten sich die Gruppen Y im Rahmen dieser Ausführungsform von Indolen und Indolinen ab, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Indole und Indoline mit zwei dieser Gruppen, insbesondere zwei Hydroxygruppen, von denen eine oder beide verethert oder verestert sein können, sind besonders bevorzugt.

Erfindungsgemäß besonders bevorzugte Gruppen Y leiten sich von Derivaten des Indolins, wie dem 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 5-Hydroxyindolin, 6-Hydroxyindolin, 5-Aminoindolin, 6-Aminoindolin und 4-Aminoindolin ab.

Ganz besonders bevorzugt sind Derivate des 5,6-Dihydroxyindolins der Formel (IVa),

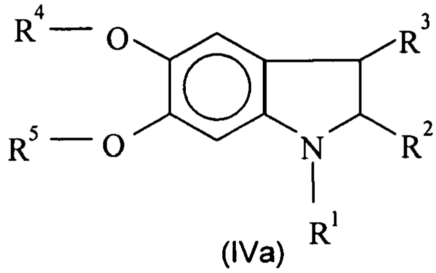

(IVa)

in der unabhängig voneinander

- $R^1$ steht für Wasserstoff, eine C1-C4-Alkylgruppe, eine C1-C4-Hydroxyalkylgruppe oder eine C3-C6-Cycloalkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine C1-C4-Alkylgruppe,
- $R^4$ steht für Wasserstoff, eine C1-C4-Alkylgruppe oder eine Gruppe -CO-$R^6$, in der $R^6$ steht für eine C1-C4-Alkylgruppe oder eine gegebenenfalls substituierte Phenylgruppe, und
- $R^5$ steht für eine der unter $R^4$ genannten Gruppen.

Erfindungsgemäß bevorzugte Vertreter sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin. Der Grundkörper, das 5,6-Dihydroxyindolin, ist ganz besonders bevorzugt.

Erfindungsgemäß bevorzugte Indole, von denen sich die Gruppe Y ableitet, sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 5-Hydroxyindol, 6-Hydroxyindol, 5-Aminoindol, 6-Aminoindol und 4-Aminoindol.

Besonders bevorzugt sind Derivate des 5,6-Dihydroxyindols der Formel (IVb),

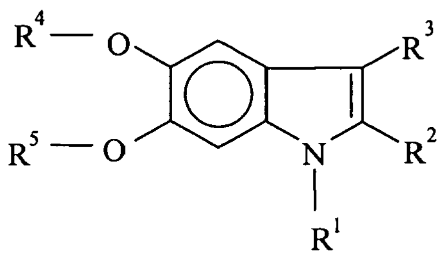

(IVb)

in der unabhängig voneinander

- $R^1$ steht für Wasserstoff, eine C1-C4-Alkylgruppe, eine C1-C4-Hydroxyalkylgruppe oder eine C3-C6-Cycloalkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff, eine C1-C4-Alkylgruppe oder eine Gruppe -$CH_2$-$NR^7R^8$, in der $R^7$ und $R^8$ unabhängig voneinander stehen für Wasserstoff oder eine C1-C4-Alkylgruppe,
- $R^4$ steht für Wasserstoff, eine C1-C4-Alkylgruppe oder eine Gruppe -CO-$R^6$, in der $R^6$ steht für eine C1-C4-Alkylgruppe oder eine gegebenenfalls substituierte Phenylgruppe, und
- $R^5$ steht für eine der unter $R^4$ genannten Gruppen.

Erfindungsgemäß bevorzugte Vertreter sind das 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol. Der Grundkörper, das 5,6-Dihydroxyindol, ist ganz besonders bevorzugt.

Gemäß einer vierten Ausführungsform der vorliegenden Erfindung leitet sich die Gruppe Y ab von einem direktziehenden Farbstoff.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Entsprechende erfindungsgemäße Mittel, die dadurch gekennzeichnet sind, daß sie mindestens einen direktziehenden Farbstoff aus der Gruppe der kationischen (basischen) Farbstoffe, vorzugsweise Basic Blue 6, C.I.-No. 51,175; Basic Blue 7, C.I.-No. 42,595; Basic Blue 9, C.I.-No. 52,015; Basic Blue 26, C.I.-No. 44,045; Basic Blue 41, C.I.-No. 11,154; Basic Blue 99, C.I.-No. 56,059; Basic Brown 4, C.I.-No. 21,010; Basic Brown 16, C.I.-No. 12,250; Basic Brown 17, C.I.-No. 12,251; Basic Green 1, C.I.-No. 42,040; Basic Orange 31; Basic Red 2, C.I.-No. 50,240; Basic Red 22, C.I.-No. 11,055; Basic Red 46; Basic Red 51; Basic Red 76, C.I.-No. 12,245; Basic Violet 1, C.I.-No. 42,535; Basic Violet 2; Basic Violet 3, C.I.-No. 42,555; Basic Violet 10, C.I.-No. 45,170; Basic Violet 14, C.I.-No. 42,510; Basic Yellow 57, C.I.-No. 12,719; Basic Yellow 87 und/oder der anionischen (sauren) Farbstoffe, und/oder der nichtionischen Farbstoffe, vorzugsweise Acid Black 1, C.L-No. 20,470; Acid Black 52; Acid Blue 7; Acid Blue 9, C.L-No. 42,090; Acid Blue 74, C.I.-No. 73,015, Acid Red 18, C.I.-No. 16,255; Acid Red 23; Acid Red 27, C.I.-No. 16,185; Acid Red 33; Acid Red 52; Acid Red 87, C.I.-No. 45,380; Acid Red 92, C.I.-No. 45,410; Acid Orange 3; Acid Orange 7; Acid Violet 43, C.I.-No. 60,730; Acid Yellow 1, C.I.-No. 10,316; Acid Yellow 10; Acid Yellow 23, C.I.-No. 19,140; Acid Yellow 3, C.I.-No. 47,005; Acid Yellow 36; D& C Brown No. 1, C.I.-No. 20,170 (Acid Orange 24); D&C Green No. 5, C.I.-No. 61,570 (Acid Green G); D&C Orange No. 4, C.I.-No. 15,510 (Acid Orange II); D&C Orange No. 10, C. I.-No. 45,425 : 1 (Solvent Red 73); D&C Orange No. 11, C.I.-No. 45,425 (Acid Red 95); D&C Red No. 21, C.I.-No. 45,380 : 2 (Solvent Red 43); D&C Red No. 27, C.I.-No. 45,410 : 1 (Solvent Red 48); D&C Red No. 33, C.I.-No. 17,200 (Acid Red 2A, Acid Red B); D&C Yellow No. 7, C. I.-No. 45,350 : 1 (Solvent Yellow 94); D&C Yellow No. 8, C.I.-No. 45,350 (Acid Yellow 73); FD& C Red No. 4, C.I.-No. 14,700 (Food Red 4); FD&C Yellow No. 6, C.I.-No. 15,985 (Food Yellow 3); Food Green 3; Pigment Red 57-1; Disperse Black 9; Disperse Blue 1; Disperse Blue 3; Disperse Violet 1; Disperse Violet 4; HC Orange 1; HC Red 1; HC Red 3; HC Red 13; HC Yellow 2; HC Yellow 4; Na-Pikramat; 1,4-Bis-(2'-hydroxyethyl)amino- 2-nitro-p-phenylendiamin; HC Yellow 5; HC Blue 2; HC Blue 12; 4-Amino-3-nitrophenol; HC Yellow 6; HC Yellow 12; 2-Nitro-1-(2'hydroxyethyl) amino-4-methylbenzol; 2-Nitro-4-amino-diphenylamin-2-carbonsäure; 2-Amino-6-chlor-4-nitrophenol; HC Red BN; 6-Nitro-1,2,3,4-tetranitrochinoxalin; o-Nitro-p-phenylendiamin; p-Nitro-m-phenylendiamin; HC Red B 54; HC Red 10; HC Red 11; HC Red 13; 2-(2'-Hydroxyethyl)amino-1-hydroxy-4,6-dinitrobenzol; 4-Ethylamino-3-nitrobenzoesäure; 2-Chlor-6-ethylamino-4-nitrophenol; 2-Hydroxy-1,4-napthochinon; 1-Propen-(4-amino-2-nitrophenyl)amin; Isatin; N-methylylisatin; HC Violet 1; HC Violet 2; 4-Nitrophenyl-aminoethylharnstoff in Mengen von 0,01 bis 25 Gew.%, vorzugsweise von 0,5 bis 10 Gew.%, insbesondere von 1 bis 5 Gew.-%, jeweils bezogen auf das gesamte Mittel, enthalten, sind bevorzugte Ausführungsformen der vorliegenden Erfindung.

Unter den vorstehend genannten Farbstoffen sind einige Vertreter besonders bevorzugt, weshalb weiter bevorzugte erfindungsgemäße Mittel, die dadurch gekennzeichnet sind, daß sie mindestens einen Direktzieher, ausgewählt aus Basic Blue 7, Basic Blue 99, Basic Violet 14, Basic Brown 16, Basic Brown 17, Basic Orange 31, Basic Red 46, Basic Red 51, Basic Red 76, Basic Yellow 57, Basic Yellow 87, Acid Black 1, Acid Blue 7, Acid Violet 43, Acid Red 23, Acid Red 52, Acid Orange 7, Acid Yellow 1, Acid Yellow 10, Acid Yellow 36, Food Green 3, Pigment Red 57-1, Disperse Black 9, Disperse Blue 1, Disperse Blue 3, Disperse Violet 1, Disperse Violet 4, HC Orange 1, HC Red 1, HC Red 3, HC Red 13, HC Yellow 2, HC Yellow 4, Na-Pikramat, 1,4-Bis-(2'-hydroxyethyl)amino-2-nitro- p-phenylendiamin, HC Yellow 5, HC Blue 2, HC Blue 12, 4-Amino-3-nitrophenol, HC Yellow 6, HC Yellow 12, 2-Nitro-1-(2'hydroxyethyl)amino-4-methylbenzol, 2-Nitro-4-amino-diphenylamin-2'-carbonsäure, 2-Amino-6-chlor-4-nitrophenol, HC Red BN; 6-Nitro-1,2,3,4-tetranitrochinoxalin, o-Nitro-p-phenylendiamin, p-Nitro-m-phenylendiamin, HC Red B 54, HC Red 10, HC Red 11, HC Red 13, 2-(2'-Hydroxyethyl)amino-1-hydroxy-4,6-dinitrobenzol, 4-Ethylamino-3-nitrobenzoesäure, 2-Chlor-6-ethylamino-4-nitrophenol, 2-Hydroxy-1,4-napthochinon, 1-Propen-(4-amino-2-nitrophenyl)amin, Isatin, N-Methylylisatin, HC Violet 1, HC Violet 2, 4-Nitrophenyl-aminoethylharnstoff in Mengen von 0,01 bis 25 Gew.%, vorzugsweise von

0,5 bis 10 Gew.%, insbesondere von 1 bis 5 Gew.-%, jeweils bezogen auf das gesamte Mittel, enthalten, bevorzugt sind.

Ferner können die erfindungsgemäßen Mittel einen kationischen direktziehenden Farbstoff enthalten. Besonders bevorzugt sind dabei

(a) kationische Triphenylmethanfarbstoffe, wie beispielsweise Basic Blue 7, Basic Blue 26, Basic Violet 2 und Basic Violet 14,

(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

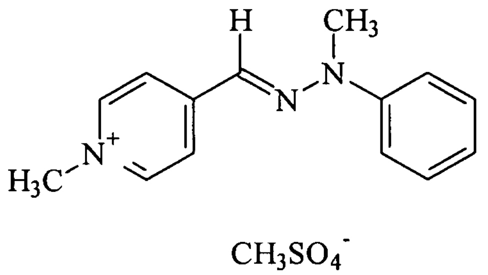

(DZ1)

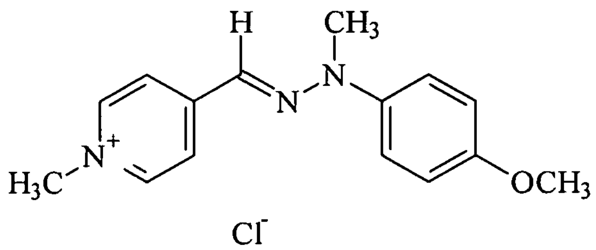

(DZ2)

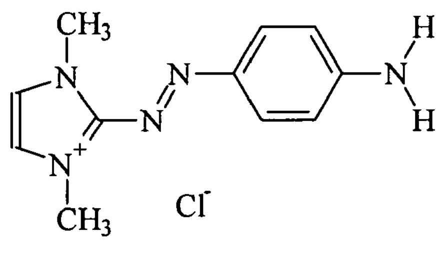

(DZ3)

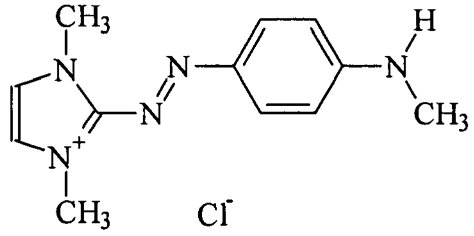

(DZ4)

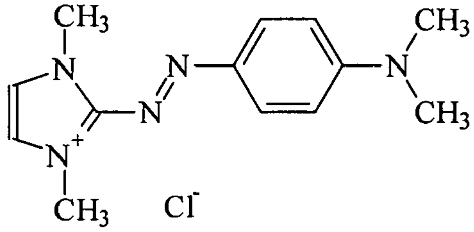

(DZ5)

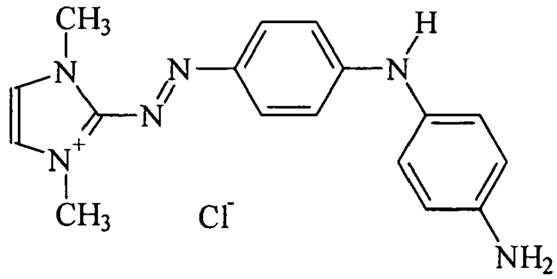

(DZ6)

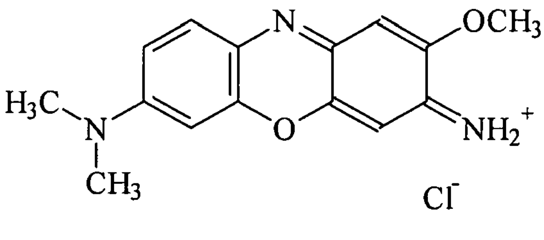

(DZ7)

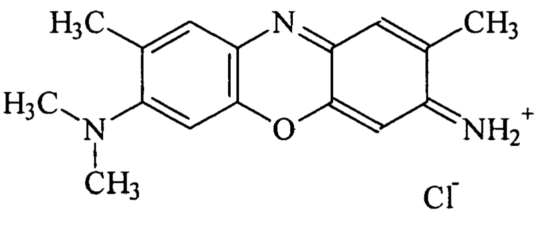

(DZ8)

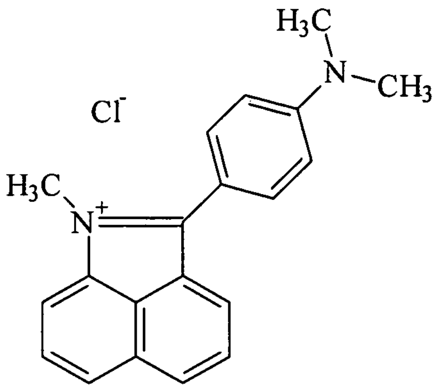

(DZ9)

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Gemäß einer ersten Ausführungsform der Erfindung steht die Gruppe S in der Strukturformel (I) für eine direkte Bindung.

In diesen Fällen wird in den meisten Fällen eine Wechselwirkung der $\pi$-Elektronen-Systeme der Gruppen X und Y auftreten, so daß in der Regel das Lichtabsorptionsverhalten des Hybridfarbstoffes sich von dem der Gruppen X und Y deutlich unterscheidet. Dadurch wird auf dem Haar ein signifikant veränderter Farbton erzielt im Vergleich zu Ausfärbungen, die mit einer Mischung entsprechender Farbstoffe durchgeführt werden, die den Gruppen X und Y entsprechen.

Ein Kerngedanke der vorliegenden Erfindung ist jedoch, die bei der Verwendung komplexer Farbstoffmischungen in vielen Bereichen, z. B. hinsichtlich Aufziehvermögen und Waschechtheit, auftretenden Probleme zu beheben, ohne den Farbton und die Nuance zu verändern.

Es ist daher in der Regel erfindungsgemäß bevorzugt, daß S für eine Spacer-Gruppe steht, über die keine Wechselwirkung der $\pi$-Elektronensysteme der Gruppen X und Y stattfindet. Bevorzugt enthält daher S mindestens ein Kohlenstoffatom mit $sp^3$-Hybridisierung auf der direkten Verbindungslinie zwischen den Gruppen X und Y.

Bevorzugte Spacergruppen S sind

- Alkylengruppen der allgemeinen Formel $-C_nH_{2n}-$, insbesondere $-(CH_2)_n-$, in denen n für eine ganze Zahl, insbesondere eine Zahl von 1 bis 8 und ganz besonders bevorzugt für eine Zahl von 1 bis 4, steht.
  Erfindungsgemäß bevorzugte Alkylengruppen sind die Methylen-, 1,2-Ethylen- und 1,3-Propylen-Gruppe,
- Cycloaliphatische Gruppen wie Cyclopentyl-, Cyclohexyl- und Cycloheptylgruppen,
- Mono- und Polyhydroxyalkylengruppen der allgemeinen Formel $-C_nH_{2n-x}(OH)_x$, in denen n für eine ganze Zahl, insbesondere eine Zahl von 1 bis 8 und ganz besonders bevorzugt für eine Zahl von 1 bis 4, steht und x für eine ganze Zahl, insbesondere für eine Zahl von 1 bis 3, steht.
  Bevorzugte Hydroxyalkylengruppen sind die Hydroxymethylen-, die Hydroxy-1,2-ethylen-, die 2-Hydroxy-1,3-Propylen, die 2,3-Dihydroxy-1,3-propylen- und die 2,3-1,4-Dihydroxybutylen-Gruppe,
- gegebenenfalls an den Alkylketten substituierte Dialkylenaminogruppen, insbesondere solche der allgemeinen Formel $-(CH_2)_n-N(Z)-(CH_2)_m-$, in der n und m unabhängig voneinander für eine ganze Zahl von 1 bis 8, insbesondere von 1 bis 4, stehen können, jedoch bevorzugt für die gleiche Zahl stehen, und Z für Wasserstoff, eine $C_{1-8}$-, insbe-

sondere $C_{1-4}$-,Alkylgruppe, eine $C_{1-8}$-, insbesondere $C_{1-4}$-, Mono-hydroxyalkylgruppe, eine $C_{2-8}$-, insbesondere $C_{2-4}$-, Dihydroxyalkylgruppe oder eine eine $C_{3-8}$-, insbesondere $C_{3-4}$-, Trihydroxyalkylgruppe steht,
sowie solche der allgemeinen Formel

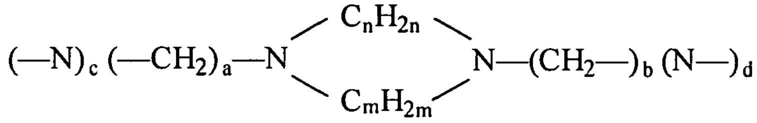

in der a und b unabhängig voneinander für ganze Zahlen von 0 bis 4 und c und d für 0 oder 1 stehen mit der Maßgabe, daß c = 0 ist, wenn a = 0 ist und d = 0 ist, wenn b = 0 ist, n für eine ganze Zahl von 1 bis 5 und m für eine ganze Zahl von 1 bis 3 steht mit der Maßgabe, daß die Summe n+m 3 bis 8 ist. Besonders bevorzugt ist die 1,4-Piperazinogruppe.

- gegebenenfalls an den Alkylketten substituierte Trialkylen-diaminogruppen, insbesondere solche der allgemeinen Formel $-(CH_2)_n-N(Z)-(CH_2)_m-N(A)-(CH_2)_p-$, in der n, m und p unabhängig voneinander für eine ganze Zahl von 1 bis 8, insbesondere von 1 bis 4, stehen können, n und m jedoch bevorzugt für die gleiche Zahl stehen, und Z und A unabhängig voneinander für Wasserstoff, eine $C_{1-8}$-, insbesondere $C_{1-4}$-,Alkylgruppe, eine $C_{1-8}$-, insbesondere $C_{1-4}$-, Mono-hydroxyalkylgruppe, eine $C_{2-8}$-, insbesondere
  $C_{2-4}$-, Dihydroxyalkylgruppe oder eine eine $C_{3-8}$-, insbesondere $C_{3-4}$-, Trihydroxyalkyl gruppe stehen,
- gegebenenfalls an den Alkylketten substituierte Ethergruppen, insbesondere solche der allgemeinen Formel $-(CH_2)_n-O-(CH_2)_m-$ , in der n und m unabhängig voneinander für eine ganze Zahl von 1 bis 8, insbesondere von 1 bis 4, stehen können, jedoch bevorzugt für die gleiche Zahl stehen,
- gegebenenfalls an den Alkylketten substituierte Polyethergruppen, insbesondere solche der allgemeinen Formel $-(CH_2)_n-O-(CH_2)_m-O-(CH_2)_m-O-(CH_2)_n-$, in der n und m unabhängig voneinander für eine ganze Zahl von 1 bis 8, insbesondere von 1 bis 4, stehen können, jedoch bevorzugt für die gleiche Zahl stehen,
- schwefelhaltige Gruppen, insbesondere Gruppen der allgemeinen Formel $-(CH_2)_n-S(O)_o-(CH_2)_m-$, in der n und m unabhängig voneinander für eine ganze Zahl von 1 bis 8, insbesondere von 1 bis 4, stehen können, jedoch bevorzugt für die gleiche Zahl stehen, und o für 0, 1 oder 2 steht.

Die Spacer S sind in den erfindungsgemäßen Hybrid-Farbstoffen über ihre beiden freien Bindungen mit den Gruppen X und Y so verknüpft, daß sie jeweils als Substituent an die Stelle eines Wasserstoffatoms der Farbstoff- bzw. Farbstoffvorprodukt-Moleküle treten, die den Gruppen X und Y zugrunde liegen.

Gemäß einer ersten bevorzugten Ausführungsform tritt die Spacergruppe S als Substituent an die Stelle eines Wasserstoffatoms, das direkt an ein Ringsystem der Gruppe X oder Y gebunden ist. Beispiele für solche Ringsysteme sind

- aromatische und cycloaliphatische Kohlenwasserstoff-Ringsysteme, insbesondere Benzol-, Naphthalin-, Anthracen-, Naphthochinon- und Anthrachinon-Systeme
- heterocyclische Ringsysteme, insbesondere Pyridin-, Pyrazol-, Pyrimidin-, Indol- und Indolinsysteme.

Gemäß einer zweiten, bevorzugten Ausführungsform tritt die Spacergruppe S als Substituent an die Stelle eines Wasserstoffatoms einer primären oder sekundären Aminogruppe, die direkt oder über eine aliphatische Kohlenwasserstoffgruppe an ein aromatisches, cycloaliphatisches oder heterocyclisches Ringsystem gebunden ist.

Gemäß einer dritten Ausführungsform tritt die Spacergruppe S als Substituent an die Stelle des Wasserstoffatoms einer Hydroxygruppe, die direkt oder über eine aliphatische Kohlenwasserstoffgruppe an ein aromatisches, cycloaliphatisches oder heterocyclisches Ringsystem gebunden ist.

Die erfindungsgemäßen Hybridfarbstoffe eignen sich hervorragend zum Färben keratinischer Fasern. Unter keratinischen Fasern sind dabei Pelze, Wolle, Federn und insbesondere menschliche Haare zu verstehen. Einer Verwendung auch auf anderen Gebieten, insbesondere in der Farbphotographie, steht aber nichts entgegen.

Ein zweiter Gegenstand der vorliegenden Erfindung sind daher Mittel zum Färben keratinischer Fasern, insbesondere menschlicher Haare, die einen Hybridfarbstoff gemäß Struktur (I) enthalten. Selbstverständlich umfaßt die erfindungsgemäße Lehre auch solche Mittel, die Kombinationen von mehr als einem Hybridfarbstoff der Struktur (I) enthalten.

Die erfindungsgemäßen Mittel zum Färben menschlicher Haare können weiterhin alle für solche Mittel üblichen Bestandteile enthalten.

Bevorzugt enthalten die erfindungsgemäßen Färbemittel mindestens einen weiteren Farbstoff, ein weiteres

Farbstoffvorprodukt und/oder ein Indol- oder Indolin-Derivat als Melanin-Vorstufe.

In einer ersten Ausführungsform sind solche Färbemittel besonders bevorzugt, die neben einem Hybridfarbstoff der Struktur (I) mindestens ein Oxidationsfarbstoffvorprodukt enthalten. Diese Oxidationsfarbstoffvorprodukte können sowohl vom Kupplertyp als auch vom Entwicklertyp sein. Bevorzugte erfindungsgemäße Mittel sind daher dadurch gekennzeichnet, daß sie weiterhin ein Oxidationsfarbstoffvorprodukt vom Kuppler- oder Entwicklertyp enthalten.

Als Oxidationsfarbstoffvorprodukte vom Entwicklertyp kommen beispielsweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate in Betracht.

Erfindungsgemäß bevorzugte Entwicklerkomponenten sind p-Phenylendiamin, p-Toluylendiamin, p-Aminophenol, 2-Chlor-4-aminophenol, o-Aminophenol, 1-(2'-Hydroxy-ethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxyethylamino-4-amino-phenol, 4,4'-Diaminodiphenylamin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 931 bzw. WO 94/08970 wie z. B. 4,5-Diamino-1-(2'-hydroxyethyl)-pyrazol. Besonders bevorzugt sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin und 4-Hydroxy-2,5,6-triaminopyrimidin.

Als Oxidationsfarbstoffvorprodukte vom Kupplertyp kommen beispielsweise m-Phenylendiaminderivate, Naphthole, Resorcin und Resorcinderivate, Pyrazolone und m-Aminophenolderivate in Betracht.

Erfindungsgemäß bevorzugte Kupplerkomponenten sind

- m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, 3-(Dimethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate wie 4-Chlor-o-aminophenol,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-3,4-diaminopyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 3,4-Methylendioxyphenol, 1-Hydroxy-3,4-methylendioxybenzol, 1-Amino-3,4-methylendioxybenzol und 1-(2'-Hydroxyethyl)-amino-3,4-methylendioxybenzol,

Erfindungsgemäß besonders bevorzugte Kuppler sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-diaminopyridin, 2,4-Diaminophenoxyethanol, 1,3-Bis-(2,4-diaminophenoxy)-propan, 2-Aminomethyl-3-amino-6-methoxypyridin und 2,6-Dihydroxy-3,4-dimethylpyridin.

Im Rahmen dieser Ausführungsform kann bevorzugt sein:

- Hybridfarbstoff der Struktur (I) mit einer Gruppe Y, abgeleitet von einem Oxidationsfarbstoffvorprodukt vom Kupplertyp, in Kombination mit mindestens einem weiteren Oxidationsfarbstoffvorprodukt vom Entwicklertyp
- Hybridfarbstoff der Struktur (I) mit einer Gruppe Y, abgeleitet von einem Oxidationsfarbstoffvorprodukt vom Entwicklertyp, in Kombination mit mindestens einem weiteren Oxidationsfarbstoffvorprodukt vom Kupplertyp
- Hybridfarbstoff der Struktur (I) mit einer Gruppe Y, abgeleitet von einem Derivat des Indols oder Indolins als Vorläufer des Melanins, in Kombination mit mindestens einem weiteren Oxidationsfarbstoffvorprodukt vom Kupplertyp

In einer zweiten Ausführungsform sind solche Färbemittel bevorzugt, die neben einem Hybridfarbstoff der Struktur (I) weiterhin mindestens einen direktziehenden Farbstoff enthalten.

Insbesondere bevorzugt sind auch erfindungsgemäße Mittel, die weiterhin ein Derivat des Indols oder Indolins als Vorläufer des Melanins enthalten.

Erfindungsgemäß bevorzugt sind solche Indole und Indoline, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Verbindungen mit zwei dieser Gruppen, insbesondere zwei Hydroxygruppen, von denen eine oder beide verethert oder verestert sind, sind besonders bevorzugt.

Erfindungsgemäß besonders bevorzugte Farbstoffvorprodukte sind Derivate des Indolins, wie das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 5-Hydroxyindolin, 6-Hydroxyindolin, 5-Aminoindolin, 6-Aminoindolin und 4-Aminoindolin.

Ganz besonders bevorzugt sind Derivate des 5,6-Dihydroxyindolins der Formel (Va),

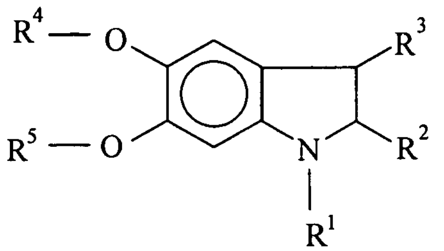

(Va)

in der unabhängig voneinander

- $R^1$ steht für Wasserstoff, eine C1-C4-Alkylgruppe, eine C1-C4-Hydroxy-alkylgruppe oder eine C3-C6-Cycloalkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine C1-C4-Alkylgruppe,
- $R^4$ steht für Wasserstoff, eine C1-C4-Alkylgruppe oder eine Gruppe -CO-$R^6$, in der $R^6$ steht für eine C1-C4-Alkylgruppe oder eine gegebenenfalls substituierte Phenylgruppe, und
- $R^5$ steht für eine der unter $R^4$ genannten Gruppen, oder ein physiologisch verträgliches Salz dieser Verbindungen mit einer organischen oder anorganischen Säure.

Erfindungsgemäß bevorzugte Vertreter sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin. Der Grundkörper, das 5,6-Dihydroxyindolin, ist ganz besonders bevorzugt.

Erfindungsgemäß bevorzugte Indole sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 5-Hydroxyindol, 6-Hydroxyindol, 5-Aminoindol, 6-Aminoindol und 4-Aminoindol.

Besonders bevorzugt sind Derivate des 5,6-Dihydroxyindols der Formel (Vb),

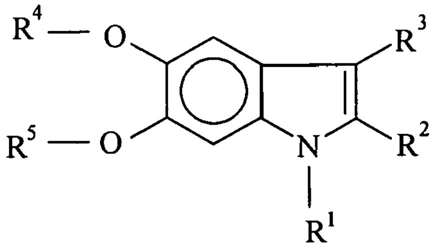

(Vb)

in der unabhängig voneinander

- $R^1$ steht für Wasserstoff, eine C1-C4-Alkylgruppe, eine C1-C4-Hydroxyalkylgruppe oder eine C3-C6-Cycloalkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff, eine C1-C4-Alkylgruppe oder eine Gruppe $-CH_2-NR^7R^8$, in der $R^7$ und $R^8$ unabhängig voneinander stehen für Wasserstoff oder eine C1-C4-Alkylgruppe,
- $R^4$ steht für Wasserstoff, eine C1-C4-Alkylgruppe oder eine Gruppe $-CO-R^6$, in der $R^6$ steht für eine C1-C4-Alkylgruppe oder eine gegebenenfalls substituierte Phenyl-gruppe, und
- $R^5$ steht für eine der unter $R^4$ genannten Gruppen, oder ein physiologisch verträgliches Salz dieser Verbindungen mit einer organischen oder anorganischen Säure.

Erfindungsgemäß bevorzugte Vertreter sind das 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol. Der Grundkörper, das 5,6-Dihydroxyindol, ist ganz besonders bevorzugt.

Selbstverständlich umfaßt die vorliegende Erfindung auch Mittel, die mehr als ein Indolin- oder Indol-Derivat oder Mischungen von Indolin- und Indol-Derivaten enthalten.

Die Indol- oder Indolin-Derivate sind in den erfindungsgemäßen Mitteln üblicherweise in Mengen von 0,05-10 Gew.-%, vorzugsweise 0,2-5 Gew.-% enthalten.

Im Rahmen dieser Ausführungsform kann bevorzugt sein:

- Hybridfarbstoff der Struktur (I) mit einer Gruppe Y, abgeleitet von einem Oxidationsfarbstoffvorprodukt vom Kupplertyp, in Kombination mit mindestens einem Derivat des Indols oder Indolins als Vorläufer des Melanins,
- Hybridfarbstoff der Struktur (I) mit einer Gruppe Y, abgeleitet von einem Oxidationsfarbstoffvorprodukt vom Entwicklertyp, in Kombination mit mindestens einem Derivat des Indols oder Indolins als Vorläufer des Melanins.

Im Rahmen der genannten Ausführungsformen ist es nicht erforderlich, daß die Oxidationsfarbstoffvorprodukte, direktziehenden Farbstoffe oder Melanin-Vorläufer jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die in den erfindungsgemäßen Mitteln enthaltenen Hybridfarbstoffe, Kuppler- und Entwicklerkomponenten mit Aminogruppen, sowie Indolin- und Indol-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bezüglich der in den erfindungsgemäßen Haarfärbe- und Tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Erfindungsgemäße Mittel, enthaltend einen Hybridfarbstoff der Struktur (I), in der sich die Gruppe Y von einem Melaninvorläufer von Indol- oder Indolin-Typ ableitet, enthalten gemäß einer bevorzugten Variante dieser Ausführungsform außer den genannten Hybridfarbstoffen keine weiteren Farbstoffe oder Farbstoffvorprodukte.

Gemäß einer weiteren bevorzugten Variante enthalten insbesondere erfindungsgemäße Mittel mit einem Hybridfarbstoff der Struktur (I), in der sich die Gruppe Y von einem Melaninvorläufer von Indol- oder Indolin-Typ ableitet, und/oder mit einem Melanin-Vorläufer weiterhin mindestens eine Aminosäure oder ein Oligopeptid.

Aminosäuren im Sinne der Erfindung sind solche Substanzen, die mindestens eine Aminogruppe und mindestens eine -COOH- oder $-SO_3H$-Gruppe aufweisen.

Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt.

Die Aminosäuren können den erfindungsgemäßen Mitteln bevorzugt in freier Form zugegeben werden. Es ist auch möglich, die Aminosäuren in Salzform einzusetzen. Bevorzugte Salze sind dann die Verbindungen mit Halogenwasserstoffsäuren, insbesondere die Hydrochloride und die Hydrobromide.

Ganz besonders bevorzugte Aminosäuren sind Lysin und insbesondere Arginin, insbesondere in freier Form, aber auch als Hydrochlorid eingesetzt.

Weiterhin können die Aminosäuren auch in Form von Oligopeptiden und Proteinhydrolysaten eingesetzt werden, wenn sichergestellt ist, daß die erforderlichen Mengen an Verbindungen, die der erfindungsgemäßen Definition der Aminosäuren entsprechen, darin enthalten sind. In diesem Zusammenhang wird auf die Offenbarung der DE-OS 22 15 303 verwiesen, auf die ausdrücklich Bezug genommen wird.

Selbstverständlich umfaßt die Erfindung auch solche Mittel, die zwei und mehr Aminosäuren oder Oligopeptide enthalten. Bevorzugt sind dabei Kombinationen von Arginin mit einer weiteren Aminosäure oder einem Oligopeptid. Die erfindungsgemäßen Mittel enthalten die Aminosäure bzw. das Oligopeptid bevorzugt in Mengen von 0,1 bis 10 Gew.-%, insbesondere 1 bis 4 Gew.-%, bezogen auf das gesamte Mittel.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine.

Gemäß einer speziellen Ausführungsform der vorliegenden Erfindung dient die Aminosäure oder das Oligopeptid nicht nur der Intensivierung der Ausfärbung, sondern übernimmt auch, zumindest teilweise, die Funktion des Alkalisierungsmittels. Im Rahmen dieser Ausführungsform werden daher bevorzugt solche Aminosäuren und Oligopeptide eingesetzt, deren 2,5 Gew.-%ige Lösungen in Wasser einen pH-Wert von 9 und größer aufweisen. Solche Aminosäuren sind die bevorzugt eingesetzten Verbindungen Arginin und Lysin. Im Rahmen dieser Ausführungsform wird das weitere Alkalisierungsmittel bevorzugt ausgewählt aus der Gruppe, die von Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxiden gebildet wird. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist im Rahmen dieser Ausführungsform bevorzugt.

Besonders vorteilhafte Eigenschaften wurden bei Mitteln gefunden, bei denen die Aminosäure oder das Oligopeptid und das weitere Alkalisierungsmittel in einem Verhältnis von 1:5 bis 5:1 vorlagen. Mengenverhältnisse von 1:2 bis 2:1 haben sich als besonders geeignet erwiesen.

In einer weiteren Ausführungsform enthält das erfindungsgemäße Mittel zusätzlich eine Kombination aus Komponente

A Verbindungen, die eine reaktive Carbonylgruppe enthalten mit Komponente

B Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden.

Erfindungsgemäße Verbindungen mit einer reaktiven Carbonylgruppe (im Folgenden auch reaktive Carbonylverbindungen oder Komponente A genannt) besitzen mindestens eine Carbonylgruppe als reaktive Gruppe, welche mit den Verbindungen der Komponente B unter Ausbildung einer beide Komponenten verknüpfenden chemischen Bindung reagiert. Ferner sind erfindungsgemäß auch solche Verbindungen als Komponente A umfaßt, in denen die reaktive Carbonylgruppe derart derivatisiert bzw. maskiert ist, daß die Reaktivität des Kohlenstoffatoms der derivatisierten bzw. maskierten Carbonylgruppe gegenüber der Komponente B stets vorhanden ist. Diese Derivate sind bevorzugt Kondensationsverbindungen von reaktiven Carbonylverbindungen mit

a)Aminen und deren Derivate unter Bildung von Iminen oder Oximen als Kondensationsverbindung
b) von Alkoholen unter Bildung von Acetalen oder Ketalen als Kondensationsverbindung.

Die Komponente A wird bevorzugt ausgewählt aus der Gruppe, die gebildet wird aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxyacetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron, Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxybenzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxybenzaldehyd, 4-Hydroxy-3,5-dimethyl-benzaldehyd, 3,5-Diethoxy-4-hydroxybenzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxybenzaldehyd, 4-Ethoxy-3-hydroxybenzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 2-Methoxy-zimtaldehyd, 4-Methoxy-zimtaldehyd, 4-Hydroxy-3-methoxy-zimtaldehyd, 3,5-Dimethoxy-4-hydroxyzimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylaminozimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1 H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon,

Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)hexa-3,5-dien-2-on, 6-(4-Pyrrolidinophenyl)hexa-3,5-dien-2-on, 5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-benzolsulfonat, -p-toluolsulfonat, -methansulfonat, -perchlorat, -sulfat, -chlorid, -bromid, -iodid, -tetrachlorozinkat, -methylsulfat-, trifluormethansulfonat, -tetrafluoroborat,

Isatin, 1-Methyl-isatin, 1-Allyl-isatin, 1-Hydroxymethyl-isatin, 5-Chlor-isatin, 5-Methoxyisatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Als CH-acide werden im allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Unter CH-acide Verbindungen fallen erfindungsgemäß auch Enamine, die durch alkalische Behandlung von quaternierten N-Heterozyklen mit einer in Konjugation zum quartären Stickstoff stehenden CH-aciden Alkylgruppe entstehen.

Die CH-aciden Verbindungen der Komponente B sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

Zusätzlich können die erfindungsgemäßen Mittel weitere Inhaltsstoffe enthalten. Ein Einsatz bestimmter Metallionen oder -komplexe kann beispielsweise bevorzugt sein, um intensive Färbungen zu erhalten. Hier sind erfindungsgemäße Mittel bevorzugt, die zusätzlich Cu-, Fe-, Mn-, Ru-Ionen oder Komplexe dieser Ionen enthalten.

Bevorzugte erfindungsgemäße Mittel enthalten 0,0001 bis 2,5 Gew.-%, vorzugsweise 0,001 bis 1 Gew.-%, bezogen auf die Gesamtzusammensetzung des Mittels, mindestens einer Verbindung aus der Gruppe Kupferchlorid ($CuCl_2$), Kupfersulfat ($CuSO_4$), Eisen(II)sulfat, Mangan(II)sulfat, Mangan(II)chlorid, Kobalt(II)chlorid, Cersulfat, Cerchlorid, Vanadiumsulfat, Kaliumjodid, Natriumjodid, Lithiumchlorid, Kaliumdichromat, Magnesiumacetat, Calciumchlorid, Cal-

ciumnitrat, Bariumnitrat, Mangandioxid ($MnO_2$) und/oder Hydrochinon.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,

- lineare Fettsäuren mit 10 bis 22 C-Atomen (Seifen),
- Ethercarbonsäuren der Formel $R-O-(CH_2-CH_2O)_x-CH_2-COOH$, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel $R-O(CH_2-CH_2O)_x-SO_3H$, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten $C_8$-$C_{22}$-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Glycerin,
- $C_8$-$C_{22}$-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel $R^1O-(Z)_x$. Diese Verbindungen sind durch die folgenden Parameter gekennzeichnet.

Der Alkylrest $R^1$ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest $R^1$ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen $R^1$

- im wesentlichen aus $C_8$- und $C_{10}$-Alkylgruppen,
- im wesentlichen aus $C_{12}$- und $C_{14}$-Alkylgruppen,
- im wesentlichen aus $C_8$- bis $C_{16}$-Alkylgruppen oder
- im wesentlichen aus $C_{12}$- bis $C_{16}$-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine $-COO^{(-)}$- oder $-SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer $C_8$-$C_{18}$-Alkyl- oder Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex® , Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat® 100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die erfindungsgemäßen Färbemittel weitere Wirk-, Hilfs- und Zusatzstoffe, wie beispielsweise

- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,

- Vitamine, Provitamine und Vitaminvorstufen, insbesondere solche der Gruppen A, $B_3$, $B_5$, $B_6$, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- onsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, $N_2O$, Dimethylether, $CO_2$ und Luft,
- Antioxidantien,

enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel enthalten die Verbindungen gemäß Formel I bevorzugt in einem geeigneten wäßrigen, alkoholischen oder wäßrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tabletten-förmige Formulierung zu integrieren.

Unter wäßrig-alkoholischen Lösungen sind im Sinne der vorliegenden Erfindung wäßrige Lösungen enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Bevorzugte erfindungsgemäße Mittel sind dadurch gekennzeichnet, daß sie zusätzlich ein nichtwässriges Lösungsmittel enthalten, wobei besonders bevorzugte erfindungsgemäße Mittel das Lösungsmittel in einer Konzentration von 0,1 - 30 Gewichtsprozent, bevorzugt in einer Konzentration von 1 - 20 Gewichtsprozent, ganz besonders bevorzugt in einer Konzentration von 2 - 10 Gewichtsprozent, jeweils bezogen auf das Mittel, enthalten.

In weiter bevorzugten erfindungsgemäßen Mitteln ist das Lösungsmittel ausgewählt aus Ethanol, n-Propanol, Isoropanol, n-Butanol, Propylenglykol, n- Butylenglykol, Glycerin, Diethylenglykolmonoethylether, Diethylenglykolmono-n- butylether , Phenoxyethanol und Benzylalkohol sowie ihren Mischungen.

Der pH-Wert der erfindungsgemäßen Mittel kann durch geeignete Inhaltstoffe wie Acidifizierungsmittel oder Alkalisierungsmittel in einem weiten Bereich eingestellt werden. Bevorzugte erfindungsgemäße Mittel sind dadurch gekennzeichnet, daß der pH-Wert des Mittels 6 - 11, bevorzugt 7,5 - 10 und besonders bevorzugt 8 -9 beträgt.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Die Gegenwart von Oxidationsfarbstoffvorprdukten ist demnach keine zwingende Voraussetzung für einen Einsatz von Oxidationsmitteln in den erfindungsgemäßen Mitteln. Als Oxidationsmittel kommen Persulfate, Chlorite und insbesondere Wasserstoffperoxid oder dessen Anlagerungsprodukte an Harnstoff, Melamin sowie Natriumborat in Frage.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.

- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Bei einer Anwendung von Oxidationsmitteln wird das eigentliche Färbemittel zweckmäßigerweise unmittelbar vor der Anwendung durch Mischung der Zubereitung des Oxidationsmittels mit der Zubereitung, enthaltend die Verbindungen der Formel I und gegebenenfalls Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40°C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann ein erfindungsgemäßes Mittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein zweiter Gegenstand der vorliegenden Erfindung ist ein Verfahren zum Färben von Keratinfasern, insbesondere menschlichen Haaren, in dem

- gewünschtenfalls ein Vorbehandlungsmittel M1 auf die Faser aufgebracht wird, dann
- ein Tönungs- oder Färbemittel M2 auf der Faser zur Anwendung kommt, wobei gewünschtenfalls dem Mittel M2 vor der Anwendung ein weiteres Mittel M3 zugegeben wird,
- dieses Färbemittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird,

wobei mindestens eines der Mittel M2, M3 oder M4 ein erfindungsgemäßes Mittel ist, d.h. mindestens eine Verbindung der Formel (I) enthält.

Die erfindungsgemäßen Mittel können demnach als Einkomponentenmittel (Tönungs- oder Färbemittel M2

bzw. Nachbehandlungsmittel M4), als Zweikomponenten Mittel (M2 + M3) oder als Dreikomponentenmittel (M2 + M3 + M4) formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Bezüglich weiterer bevorzugter Ausführungsformen der erfindungsgemäßen Verfahren gilt mutatis mutandis das zu den erfindungsgemäßen Mitteln Gesagte.

Weitere Gegenstände der vorliegenden Erfindung sind die Verwendung eines erfindungsgemäßen Hybridfarbstoffes zum Färben keratinischer Fasern, insbesondere menschlicher Haare sowie die Verwendung eines erfindungsgemäßen Mittels zum Färben keratinischer Fasern, insbesondere menschlicher Haare.
Bezüglich bevorzugter Ausführungsformen dieser erfindungsgemäßen Verwendungen gilt mutatis mutandis das zu den erfindungsgemäßen Mitteln Gesagte.

**Patentansprüche**

**1.** Hybridfarbstoff, insbesondere zum Färben keratinischer Fasern, der Struktur (I),

$$X - S - Y \quad (I)$$

in der X steht für eine Gruppe, die abgeleitet ist von

- Indolin-2,3-dion (Isatin) oder substituierten, insbesondere N-substituierten Indolin-2,3-dionen (Isatinen),
- 2-Oxoessigsäure oder 2- Oxoessigsäurederivaten,
- Tropolon oder Tropolonderivaten,
- ungesättigten Aldehyden;

Y steht für eine Gruppe, die abgeleitet ist von

- einem Oxidationsfarbstoffvorprodukt vom Kuppler- oder Entwicklertyp,
- einem Derivat des Indols oder Indolins als Vorläufer des Melanins oder
- einem direktziehenden Farbstoff, und

S steht für eine direkte Bindung oder eine Spacer-Gruppe
mit der Maßgabe, daß S nicht für eine Alkylen-, Mono- oder Poly-hydroxyalkylengruppe steht, wenn Y von einem direktziehenden Farbstoff abgeleitet ist.

**2.** Hybridfarbstoff gemäß Anspruch 1, **dadurch gekennzeichnet, daß** X steht für eine Gruppe, die ausgewählt ist aus

- Indolin-2,3-dion,
- 1-(4-Nitrophenyl)indolin-2,3-dion,
- 1-(2-Nitrophenyl)indolin-2,3-dion,
- 1-(4-Amino-2-nitrophenyl)indolin-2,3-dion,
- 1-(5-Nitro-2-pyridyl)indolin-2,3-dion,
- 1-Hydroxymethylisatin,
- 1-Hydroxymethyl-5-methylisatin,
- 1-Hydroxymethyl-5-chlorisatin,
- 1-Hydroxymethyl-5-sulfoisatin,
- 1-Hydroxymethyl-5-carboxyisatin,
- 1-Hydroxymethyl-5-nitroisatin,
- 1-Hydroxymethyl-5-bromisatin,
- 1-Hydroxymethyl-5-methoxyisatin,
- 1-Hydroxymethyl-5,7-dichlorisatin,
- 1-Dimethylaminomethylisatin,
- 1-Diethylaminomethylisatin,
- 1-(2,3-Dihydroxypropyl)isatin,
- 1-(Bis-(2-hydroxyethyl)-aminomethyl)-isatin,
- 1-(2-hydroxyethylaminomethyl)-isatin,
- 1-(Bis-(2-hydroxypropyl)-aminomethyl)-isatin,
- 1-Pyrrolidinomethylisatin,

- 1-Piperidinomethylisatin,
- 1-Morpholinomethylisatin,
- 1-(2-Morpholinoethyl)isatin,
- 1-(1,2,4-Triazolyl)-methylisatin,
- 1-(1-Imidazolyl)-methylisatin,
- 1-Carboxymethylaminomethylisatin,
- 1-(2-Carboxyethylaminomethyl)-isatin,
- 1-(3-Carboxypropylaminomethyl)-isatin,
- 1-(Bis-(2-hydroxyethyl)-aminomethyl)-5-methylisatin,
- 1-Piperidinomethyl-5-chlorisatin,
- 1-(2-Sulfoethylaminomethyl)-isatin,
- 1-(2-Sulfoethyl)isatin,
- 1-(3-Sulfopropyl)isatin,
- 1-Allylisatin,
- 1-(2-Dimethylamino)isatin,
- 1-(2-Pyrrolidino)isatin,
- 1-(2-Piperidino)isatin,
- N-(2-furylmethyl)isatin,
- 1-(2-Thienylmethyl)isatin,
- 1-(2-, N- (3- oder N-(4-Pyridylmethyl)-isatin,
- 1-Allylisatin-5-sulfonsäure,
- 5-Chlorisatin,
- 5-Methyl-N-(hydroxyethyl)isatin,
- 5,7-Dichlorisatin,
- 5-Nitro-N-allylisatin

**3.** Hybridfarbstoff gemäß Anspruch 1, **dadurch gekennzeichnet, daß** X steht für eine Gruppe, die ausgewählt ist aus

- 2-Oxoessigsäure,
- 2-(2-Aminophenyl)-2-Oxoessigsäure,
- 2-(2-Amino-5-methylphenyl)-2-Oxoessigsäure,
- 2-(2-Amino-5-chlorphenyl)-2-Oxoessigsäure,
- 2-(2-Amino-5- nitrophenyl)-2-Oxoessigsäure,
- 2-(2-Amino- 5-methoxyphenyl)-2-Oxoessigsäure,
- 2-(2-Hydroxylaminophenyl)-2-Oxoessigsäure,
- 2-(2-(2-Hydroxylethylamino)-phenyl)-2-Oxoessigsäure,
- 2-(2-Benzylaminophenyl)-2-Oxoessigsäure,
- 2-(2-(2- Sulfoehtylamino)-phenyl)-2-Oxoessigsäure,
- 2-(2-(3-Sulfopropylamino)-phenyl)-2-Oxoessigsäure,
- 2-(2- Allylaminophenyl)-2-Oxoessigsäure,
- 2-(2- Carboxymethylaminophenyl)-2-Oxoessigsäure,
- 2-(2-Amino-5- bromphenyl)-2-Oxoessigsäure,
- 2-(2-Amino-5,7-dichlorphenyl)-2-Oxoessigsäure,
- 2-(2-Amino-5-sulfophenyl)-2-Oxoessigsäure,
- 2-(2-Amino-5-carboxyphenyl)-2-Oxoessigsäure,
- 2-(2-Amino-4-carboxyphenyl)-2-Oxoessigsäure,
- sowie Natrium-, Kalium-, Ammonium- oder Calciumsalze der vorstehend genannten Verbindungen.

**4.** Hybridfarbstoff gemäß Anspruch 1, **dadurch gekennzeichnet, daß** X steht für eine Gruppe, die ausgewählt ist aus Tropolonen der Formel II

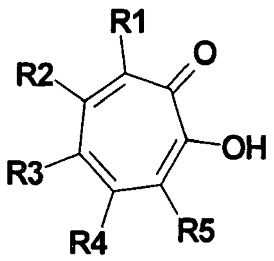

(II)

in der $R^1$, $R^2$, $R^3$, $R^4$, und $R^5$ ein Wasserstoff-, ein Halogenatom oder eine $(C_1\text{-}C_4)$-Alkylgruppe bedeuten, wobei die Reste $R^1$ und $R^2$ auch gemeinsam einen an die Doppelbindung ankondensierten Benzolring, der seinerseits durch Hydroxy-, $(C_{1\text{-}}C_4)$-Alkyl-, $(C_1\text{-}C_4)$-Alkoxygruppen oder Halogenatome substituiert sein kann, bilden können, und
wobei folgende Verbindungen besonders bevorzugt sind:

- Tropolon,
- Purpurogallin,
- der Methylether von Purpurogallin,
- 3,5,7-Tribromtropolon,
- 3,5,7-Trimethyltropolon,
- 7-(2-Propyl)-tropolon.

**5.** Hybridfarbstoff gemäß Anspruch 1, **dadurch gekennzeichnet, daß** X steht für eine Gruppe, die ausgewählt ist aus ungesättigten Aldehyden beschrieben durch die tautomeren Grenzformeln IIIa bzw. IIIb,

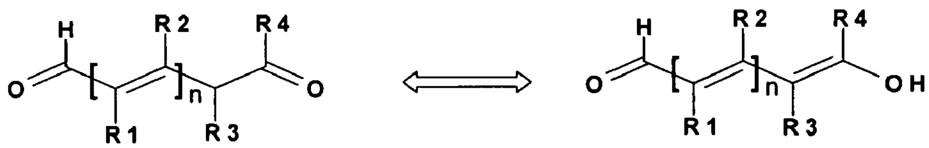

(IIIa) (IIIb)

in der $R^1$, $R^2$, $R^3$ und $R^4$ unabhängig voneinander für Wasserstoff, Halogen, einen $C_{1\text{-}}C_4$-Alkoxy-, $C_1$-$C_4$-Alkyl-, Aryl- oder $C_1$-$C_4$-Alkoxy-$C_1$-$C_4$-Alkylrest und n für die Zahlen 1 oder 2 stehen, wobei $R^1$ und $R^2$, $R^1$ und $R^3$, $R^2$ und $R^3$ sowie $R^2$ und $R^4$ jeweils zusammen mit dem Restmolekül einen 5- bis 7-gliedrigen Ring bilden können, wenn n gleich 1 ist, sowie den entsprechenden Mono-, Bis- bzw. ω-Alkoxyacetalen, wobei folgende Verbindungen besonders bevorzugt sind:

- Glutaconaldehyd,
- 1-Formyl-3-hydroxymethylen-l-cyclohexen,
- 1-Formyl-3-hydroxymethylen-1-cyclohepten,
- 7-Hydroxy-2,4,6-heptatrienal
- substituierten Derivate sowie physiobgisch verträgliche Salze der vorstehend genannten Verbindungen, insbesondere Alkali- und Ammoniumsalzen, wobei die Tetrabutylammoniumsalze und Na-Salze sowie deren Gemische besonders bevorzugt sind.

**6.** Mittel zum Färben keratinischer Fasern, **dadurch gekennzeichnet, daß** es einen Hybridfarbstoff gemäß einem der Ansprüche 1 bis 5 enthält.

**7.** Mittel nach Anspruch 6, **dadurch gekennzeichnet, daß** es weiterhin ein Oxidationsfarbstoffvorprodukt vom Kuppler-

oder Entwicklertyp enthält.

8. Mittel nach Anspruch 6 oder 7, **dadurch gekennzeichnet, daß** es weiterhin einen direktziehenden Farbstoff enthält.

9. Mittel nach einem der Ansprüche 6 bis 8, **dadurch gekennzeichnet, daß** es weiterhin ein Derivat des Indols oder Indolins als Vorläufer des Melanins enthält.

10. Verwendung eines Hybridfarbstoffes nach einem der Ansprüche 1 bis 5 zum Färben keratinischer Fasern, insbesondere menschlicher Haare.

11. Verwendung eines Mittels nach einem der Ansprüche 6 bis 9 zum Färben keratinischer Fasern, insbesondere menschlicher Haare.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 1530229 B
- DE 19930927 A1
- EP 0740931 A
- WO 9408970 A
- EP 998908 A2
- DE 2215303 A
- DE 3725030 A
- DE 3723354 A
- DE 3926344 A

### In der Beschreibung aufgeführte Nicht-Patentliteratur

- **CH. ZVIAK.** *The Science of Hair Care,* vol. 7, 264-267
- *Dermatology,* 1986
- Dermatology. Verlag Marcel Dekker Inc, 1986
- **CH. ZVIAK.** *The Science of Hair Care,* 248-250
- *Oxidationsfarbstoffvorprodukte,* vol. 8, 264-267
- Dermatology. Verlag Marcel Dekker Inc, vol. 7
- **KH. SCHRADER.** Grundlagen und Rezepturen der Kosmetika. Hüthig Buch Verlag, 1989